# EUROPEAN PATENT APPLICATION

(11) **EP 1 676 601 A1**
(43) Date of publication of application: **05.07.2006**
(21) Application number: 05257961.2
(22) Date of filing: 22.12.2005
(51) Int. Cl.: A61N 2/00, A61B 17/22

(54) **System for treating a thrombus in a blood vessel**

(30) Priority: 29.12.2004 US 25205
(71) Applicant: Nitinol Development Corporation, Fremont, California 94539 (US)
(72) Inventor: Meretei, Attila, Fremont, CA 94538 (US)
(74) Representative: Belcher, Simon James

(57) **Abstract**

Ferrofluids having magnetic particles dispersed therein are introduced locally via a catheter, a micro-catheter, or intravenously, to the bloodstream of a patient. The ferrofluids are magnetically manipulated or moved throughout the blood vessels of the patient by an external magnetic field generator until the intended occluding thrombus is broken up and removed. The external magnetic field generator, which can be stationary or portable, creates a vortex, high velocity jets, or other motion, within the ferrofluids, by rotating or moving at least one magnet (21) relative to the patient. The at least one magnet is provided in the magnetic field generator. The vortex, high velocity jets, or other motion of the ferrofluids are used to break-up and remove the occluding thrombus. Drugs or abrasive particles, or both, may be incorporated with the ferrofluids and delivered to the bloodstream with the ferrofluids to help break-up and remove the occluding thrombus as well.

## Description

The invention generally relates to systems and methods for treating a thrombus in the bloodstream of a patient. More specifically, the invention relates to systems and methods for treating a thrombus in a blood vessel by delivering and manipulating ferrofluids in the bloodstream of the patient.

Occlusions within blood vessels prevent blood from flowing to areas of organs that are intended to be supplied with oxygen from the vessels. Ischemic stroke, for instance, is a disease in which locally formed thrombus or embolic material occludes a cerebral artery within the brain. An occlusion within the artery may prevent blood flow to the area of the brain that is ordinarily to be supplied with oxygen from the artery. Insufficient oxygenation, or hypoxia, results. Such hypoxia leads to neuronal death and function loss. If critical parts of the brain are damaged by hypoxia, the stroke may lead to death.

In the past, thrombolytic drugs have been infused into an occluded vessel in order to break up the occluding thrombus. A micro-catheter is introduced into the occluded vessel to infuse the thrombolytic drugs into the vessel. Thrombylosis is then induced by the infused drug, and ideally the occluding thrombus within the vessel disintegrates to permit blood flow through the vessel.

Alternatively, mechanical means have been used to break up and remove an occluding thrombus from a vessel. Such mechanical means may include a means of capturing or cutting and retracting the thrombus, a vacuum or suction means, or a means of macerating the thrombus wherein the re-established blood flow washes away the macerated thrombus. Still other mechanical means for breaking up and removing a thrombus include ultrasound, laser or fluid-jet techniques.

While the infusion of thrombolytic drugs into a blood vessel using a micro-catheter is relatively easy and does not require extensive manipulation or dexterity by the medical professional, it may take hours for the thrombolytic drug to take effect. Further, the thrombolytic drug may not mix well with the thrombus material resulting in the thrombus not being effectively broken up as quickly as desired or at all, in some instances. Further still, infusion of the thrombolytic drugs often causes bleeding elsewhere in the body.

With respect to the various alternative mechanical means for breaking up and removing a thrombus, fairly quick and sometimes immediate relief from the thrombus is achieved, and bleeding in other body parts is avoided due to drug side effects as no thrombolytic drugs are typically used. However, such mechanical means are often difficult to navigate with precision as is often required with delicate vessels such as those in the vasculature of a brain. Moreover, navigation and operation of the mechanical means typically require great manual dexterity and experience by the medical professional in order not to cause unintended harm, such as intracranial bleeding, to a patient.

In view of the above, a need exists for systems and methods that more easily and safely treat a thrombus in a blood vessel.

The systems and methods of the invention introduce ferrofluids into the bloodstream of a patient and magnetically manipulate the ferrofluids in order to break up and remove a thrombus therewithin. In some embodiments of the systems and methods of the invention, the ferrofluids are introduced locally to a targeted blood vessel using a catheter. More specifically, a microcatheter is used for delivering ferrofluids locally to smaller vessels, whereas a larger catheter is used for delivering ferrofluids locally to larger vessels within a patient. The ferrofluids are then magnetically manipulated to remove the occluding thrombus. In other embodiments, the ferrofluids are intravenously introduced to the bloodstream of the patient. The intravenously introduced ferrofluids are magnetically moved to the occluding thrombus site and then further magnetically manipulated to break-up and remove the occluding thrombus.

In some embodiments, the ferrofluids incorporate abrasive particles that are manipulated along with the ferrofluids to breakup the occluding thrombus in the bloodstream. In other embodiments, the ferrofluids incorporate thrombolytic drugs that are manipulated along with the ferrofluids to breakup the occluding thrombus in the bloodstream. In still other embodiments, the ferrofluids incorporate a combination of abrasive particles and a thrombolytic drug that are manipulated along with the ferrofluids to breakup the occluding thrombus in the bloodstream.

In those embodiments where a microcatheter is used to introduce the ferrofluids into the bloodstream, the microcatheter may include at least one sensor at its tip or at a tip of a guidewire used with the microcatheter, for example. The at least one sensor helps identify conditions pertaining to the thrombus site and the movement of the ferrofluids in particular. To this end, the at least one sensor may identify conditions such as pressure, temperature, flow, shaft deflection or the like to indicate how the ferrofluids are acting at the thrombus site within the occluded blood vessel. Based on the data sensed by the at least one sensor, the magnetic field, or the position of the patient relative to the magnetic field, may be altered in order to more appropriately manipulate or move the ferrofluids to breakup and remove the occluding thrombus.

The systems and methods of the invention further provide a magnetic field generator. The magnetic field generator is external of the patient and is used to manipulate or move the ferrofluids within the bloodstream of the patient. The magnetic field generator may induce a vortex, a high velocity jet or other movement in the ferrofluids introduced in into the vasculature of the patient. The magnetically induced vortex, high velocity jet or other movement, breaks up and removes the occluding thrombus in a targeted blood vessel. After the thrombus is removed, the ferrofluids may remain in the patient for eventual consumption by naturally occurring phagocytotic cells, or the magnetic components of the ferrofluids may be magnetically recaptured and removed from the bloodstream using the catheter or microcatheter, for example.

In some embodiments, the magnetic field generator comprises a tubular member into which the patient is placed. The magnetic field generator in this instance is similar to an MRI or CT scanner system, whereby the patient lies prone on a movable table that is transportable into and out of the tubular member. The tubular member according to this embodiment of the systems and methods of the invention further comprises a movable collar having at least one magnet circumferentially arranged about a portion of the tubular member. The collar of at least one magnet surrounds the body part of the patient having the occluding thrombus. When stationary, the collar of at least one magnet provides a magnetic field sufficient to concentrate intravenously delivered ferrofluids at the intended thrombus site. When rotated, or otherwise moved in relation to the patient's body, the collar of at least one magnet provides a changing magnetic field sufficient to induce the vortex, high velocity jet or other movement, of the ferrofluids at the occluding thrombus site that is used to break-up and remove the occluding thrombus. Of course, independent magnets could instead be used to concentrate the intravenously delivered fluids at the intended thrombus site.

In still other embodiments, the magnetic field generator is a more portable system transportable in emergency vehicles, for example. The portable magnetic field generator comprises a smaller scale tubular member into which the body part having the occluding thrombus is placed. The portable tubular member also comprises a movable collar having at least one magnet that surrounds the occluding thrombus site of the patient when the body part is placed within the portable tubular member. As in the larger scale tubular member, when stationary the collar of at least one magnet provides a sufficient magnetic field to concentrate the intravenously delivered ferrofluids at the intended thrombus site, whereas rotation, or other (e.g., longitudinal) movement, of the collar of at least one magnet provides a sufficient changing magnetic field to induce the vortex, high velocity jets or other movement, of the ferrofluids that break-up and remove the thrombus similar to as described above. The portable tubular member is ideally sufficiently lightweight that it can be managed by a single emergency or other medical professional and placed around the intended body part with minimal movement of the patient.

The systems and methods of the invention thus provide a low profile delivery system for delivering ferrofluids to the bloodstream, whereby the ferrofluids are easily manipulated within a vessel. Rigid mechanical components are not required to be introduced into the bloodstream or to penetrate through the occluding thrombus in a blood vessel. Unintended damage to blood vessels or other organs is minimized as a result.

The systems and methods of the invention simplify the treatment of thrombus occlusions and can require less training for a medical professional administering the ferrofluids to a patient. Where the ferrofluids are introduced intravenously to the bloodstream of a patient, emergency medical personnel or front-line hospitals, rather than specialized stroke and neuro-vascular oriented medical centers, may more easily administer the ferrofluids to a patient. The ready access of such emergency and front-line hospitals equipped with the systems and methods of the invention can minimize the detrimental impact an occluding thrombus can have on a patient by reducing the time that elapses between the onset or diagnosis of an occlusion and the resolution of the occlusion. Moreover, in the case of a thrombus induced stroke, the ready access of facilities equipped with the systems and methods of the invention can also increase substantially the recovery prospects for one who has suffered from an occluding thrombus induced stroke. Thus, the systems and methods of the invention provide a safer, simpler, and easier manner of treating an occluding thrombus within a blood vessel of a patient.

The system of the present invention can be used in a method for treating an occluding thrombus in a blood vessel, comprising: delivering ferrofluids having magnetic particles disposed therein to an occluding thrombus within a blood vessel; and magnetically manipulating the ferrofluids to remove the thrombus.

The above and other features of the invention, including various novel details of construction and combinations of parts, will now be more particularly described with reference to the accompanying drawings and claims. It will be understood that the various exemplary embodiments of the invention described herein are shown by way of illustration only and not as a limitation thereof. The principles and features of this invention may be employed in various alternative embodiments without departing from the scope of the invention.

Embodiments of the invention will now be describe by way of example with reference to the accompanying drawings, in which:
Figure 1 illustrates a blood vessel with an occluding thrombus therein;
Figures 2 illustrates an area of the brain in which an occluding thrombosis may occur;
Figure 3 illustrates lower limbs in which an occluding thrombosis may occur;
Figures 4a-4c illustrate various ferrofluid particles and ferrofluids according to the invention;
Figure 5 illustrates a micro-catheter introducing ferrofluids locally to a blood vessel in the brain of a patient according to the invention;
Figures 6a-6c illustrate various stages of the vortex created by the ferrofluids breaking-up an occluding thrombus according to the invention;
Figure 7 illustrates a magnetic field generator according to one aspect of the invention;
Figure 8 illustrates a patient subjected to the magnetic field generator of Fig. 7 according to the invention;
Figure 9 illustrates a portable magnetic field generator according to another aspect of the invention;
Figure 10 illustrates a patient subject to the magnetic field generator of Fig. 9 according to the invention;
Figure 11 illustrates micro-catheter guide wire tip having sensors incorporated therewith according to another aspect of the invention;

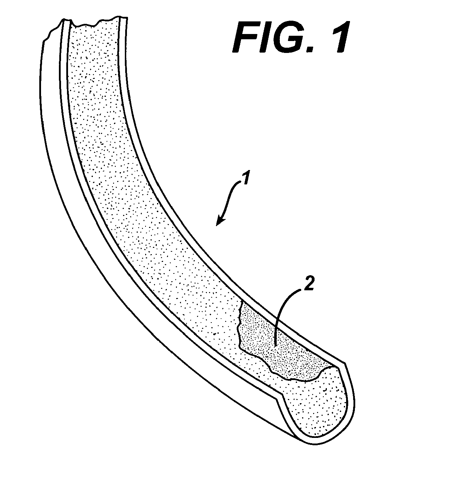

Fig. 1 illustrates an exemplary blood vessel 1 of the vasculature of a human anatomy. The blood vessel 1 is shown as having an occluding thrombus 2 extending from a sidewall of the blood vessel. The occluding thrombus 2 narrows the blood-flow passageway through the blood vessel 1 resulting in hypoxia, stroke or other undesirable maladies and conditions. The occluding thrombus 2 may occur anywhere in the vasculature of the human anatomy, but is often formed in the blood vessels of the brain and the lower limbs of a patient.

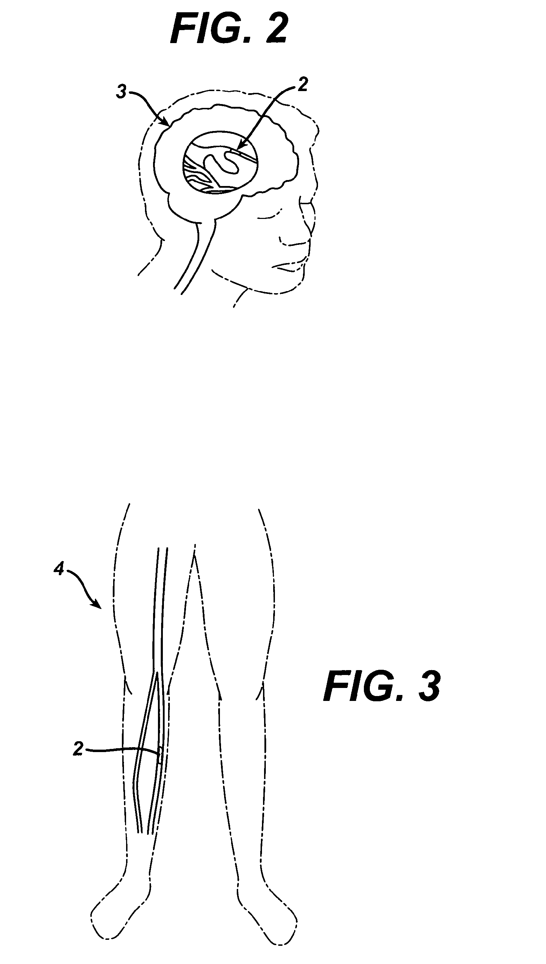

Fig. 2 illustrates a thrombus 2 located in blood vessels of the brain 3, for example. Fig. 3 illustrates a thrombus 2 located in blood vessels of the leg 4, for example. The systems and methods for treating an occluding thrombus according to the invention may be applied to treat an occluding thrombus located anywhere in the vasculature of the human anatomy. The artisan should readily appreciate therefore that the occluding thrombus shown and described herein with respect to either of the brain and lower limbs are for illustrative purposes only. Similar systems and methods may be used to treat an occluding thrombus at other locations in the vasculature of a patient as well using the systems and methods of the invention.

Figs. 4a-4c illustrate various configurations of a ferrofluid according to the invention. The ferrofluids may be colloids with magnetic particles of nanometer or micron size dispersed in a carrier fluid. The carrier fluid may be water, water-alcohol mixtures, or a variety of hydrocarbons such as, paraffin oil or synthetic esters, for example. The magnetic particles may be comprised of materials such as magnetite or cobalt ferrite, for example. The artisan should appreciate that other materials having similar properties as known in the art are also readily usable as the carrier fluids or magnetic particles, as appropriate, with the systems and methods of the invention.

Fig. 4a more specifically illustrates a single magnetic particle 10 according to the invention. A plurality of magnetic particles 10 in colloidal solution within a carrier fluid comprises the ferrofluids that are delivered to the bloodstream of a patient to break-up and remove an occluding thrombus according to the invention. Each single magnetic particle 10 is preferably 5-500 nanometers in size and is preferably comprised of bio-compatible material of sufficient radiopacity as to be visible using known fluoroscopy techniques, for example. However, non-radiopaque magnetic particles may also be used if the ferrofluid in the vasculature is visualized by means other than fluoroscopy. Of course, the artisan will appreciate that the ferrofluids may also comprise a combination of different magnetic particles 10 dispersed within the colloid.

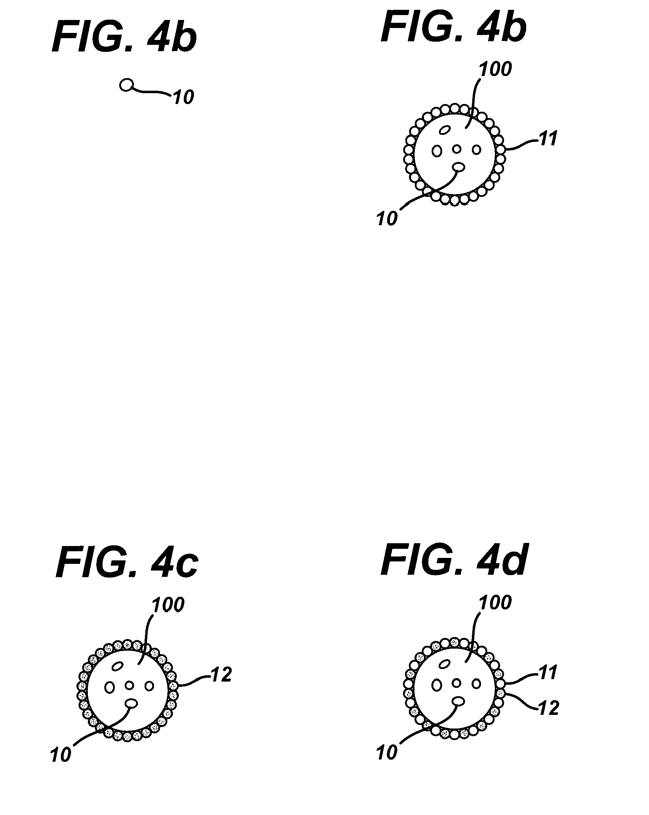

Fig. 4b illustrates a ferrofluid droplet 100 having magnetic particles 10 dispersed therein and having particles of an abrasive material 11 added to the ferrofluid. The abrasive particles 11 are thus added to the ferrofluid colloid. The abrasive particles 11 will be displaced to the surface of the ferrofluid droplet 100 (as shown in Fig. 4b) once the ferrofluid droplet 100 is exposed to a sufficient magnetic field. Other abrasive particles may also be expelled to the surface of the ferrofluid droplets because of surface characteristics. Such surface characteristics may include hydrophobic functional groups that would expel the abrasive particle to the surface of a ferrofluid droplet having a hydrophilic carrier fluid. Alternately, abrasive particles that have hydrophilic functional groups on their surface would be expelled to the surface of a ferrofluid droplet having a hydrophobic carrier fluid. The abrasive material may comprise a bio-compatible abrasive material such as beads made of glass, polymers, or non-magnetic metals such as silver, platinum or gold, or any other bio-compatible abrasive material known in the art that can be delivered with the ferrofluid droplet 100.

Fig. 4c illustrates a ferrofluid droplet 100 having magnetic particles 10 dispersed therein and being impregnated with a thrombolyitic drug, some of which may be expelled to the surface of the ferrofluid droplet. The thrombolyitic drug may comprise tPA, for example, or other thrombolytic drug known in the art. The thrombolytic drug may be bound to the surface of the ferrofluid droplets 100, as shown in Fig. 4c, or may be bound to the surface of the magnetic particles 10 dispersed within the ferrofluid, bonded to the abrasive particles 11 dispersed within the ferrofluids, carried in pores or internal cavities of the magnetic particles 10, mixed into the carrier fluids, or may be added to the colloid of ferrofluids via carrier particles 12 (Fig. 4d) designed to carry the drug within the colloid of ferrofluids.

Fig. 4d illustrates a ferrofluid droplet 100 containing carrier particles 12 that carry the thrombolytic drug. As shown also in Fig. 4d, the ferrofluid droplet 100 also contains magnetic particles 10 and the abrasive particles 11, which have been displaced to the surface of the ferrofluid droplet 100 after exposure to a magnetic field according to the systems and methods of the invention.

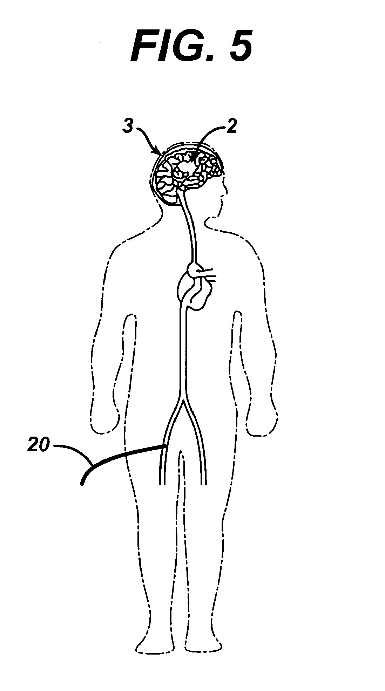

In practice, ferrofluids are delivered to the site of the occluding thrombus in conventional manner using a catheter, a micro-catheter, or intravenously. A larger catheter is used to deliver ferrofluids locally to larger vessels, whereas a micro-catheter is used to deliver ferrofluids locally to smaller vessels. As shown in Fig. 5, wherein the micro-catheter 20 is used to deliver ferrofluids to the brain 3, for example, the ferrofluids are generally delivered directly to the site of the occluding thrombus 2. On the other hand, when intravenous delivery of the ferrofluids is used (not shown), the ferrofluids are delivered systemically to the bloodstream of the patient and then subsequently magnetically manipulated to the site of the occluding thrombus according to the invention.

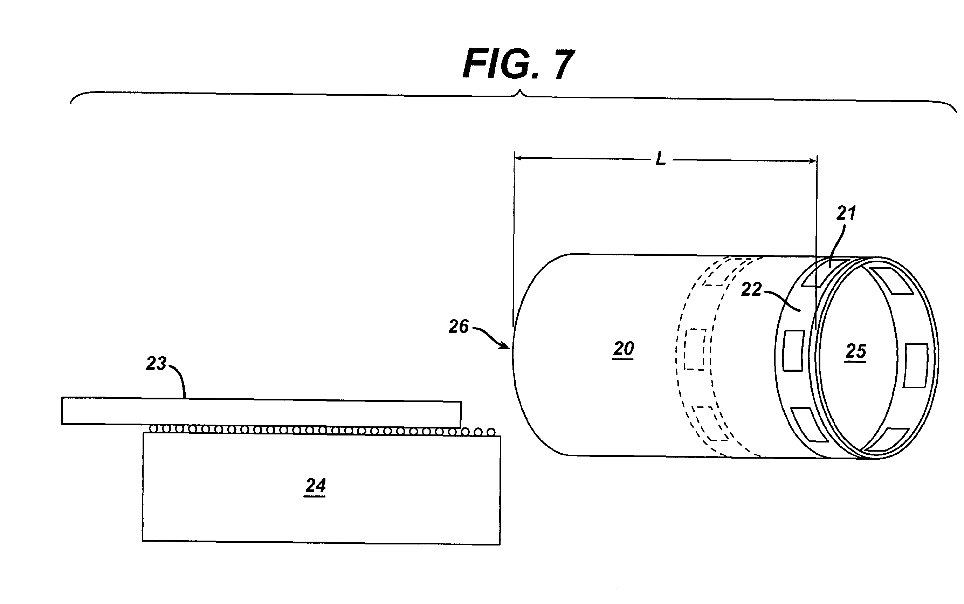

Once the ferrofluids are located at the site of the occluding thrombus, whether by direct catheter delivery or by indirect intravenous delivery with subsequent magnetic manipulation, the ferrofluids are then subjected to a magnetic field generated by an external magnetic field generator, as will be discussed in more detail below with respect to Figs 7-10. The external magnetic field generator externally encircles the body part whereat the occluding thrombus site is located. By rotating or otherwise moving the magnetic field, the ferrofluids within the blood vessel of the encircled body part are also rotated or moved.

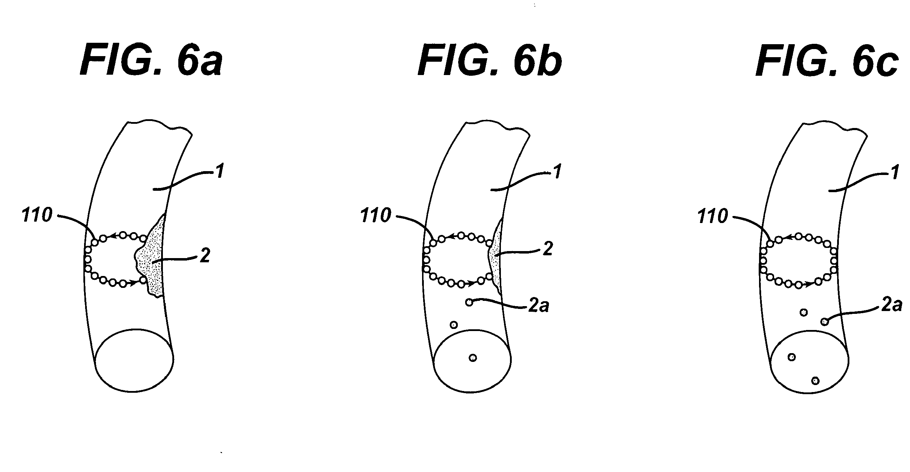

Figs. 6a-6c illustrate various stages of rotating the ferrofluids at the site of an occluding thrombus 2 within a blood vessel 1. For example, Fig. 6a illustrates the ferrofluid being rotated in the blood vessel 1. By rotating the external magnetic field sufficiently the ferrofluids are induced to form a vortex 10 within the blood vessel 1. Ideally, as shown in Figs. 6a-6c, the ferrofluid vortex 110 rotates along the inner surface of the blood vessel 1 to break-up or otherwise detach the occluding thrombus 2 from the blood vessel 1. The ferrofluids may instead be induced by the magnetic field to produce high velocity jets or other movement to break-up and remove the occluding thrombus, although the inducement and use of the vortex 110 is described in greater detail herein. In Fig. 6a, for example, the ferrofluid vortex 110 has just begun and the occluding thrombus 2 is largely intact. In Fig. 6b, the ferrofluid vortex 110 has taken place for awhile and has broken-up or otherwise detached portions of the occluding thrombus 2 from the blood vessel 1. Remnants 2a of the broken-up thrombus 2 are evident in Fig. 6b, for example. Ideally, the remnants 2a are generally dispersed through the blood vessel 1 without event. In Fig. 6c the occluding thrombus 2 has been removed by the ferrofluid vortex 110, and only the remnants 2a of the thrombus remain for dispersal through the bloodstream. Although the rotation of the ferrofluid vortex 110 shown in Figs. 6a-6c is counterclockwise, the artisan will appreciate that clockwise, or other, rotational directions are also usable according to the systems and method of the invention.

Where the ferrofluid droplets 100 used have magnetic particles 10 but are devoid of abrasives 11 or thrombolytic drugs 12, the ferrofluid vortex 110 may break-up the occluding thrombus 2 or may simply detach the occluding thrombus 2 from the blood vessel 1, rather than fully breaking up the occluding thrombus. Thereafter, the detached thrombus 2 may be mechanically extracted from the blood vessel using a catheter, a micro-catheter, or other mechanical thrombus removal device in conventional manner. The risks of the additional extraction procedure are self-evident, but such a drug-less procedure minimizes the bleeding that often occurs in unintended areas when thrombolytic drugs are used to treat a thrombus.

Where the ferrofluid droplets 100 have magnetic particles 10 and abrasive particles 11 dispersed therein, the abrasive particles 11 work in combination with the vortex 110 to break-up and remove the occluding thrombus 2, ideally without need for mechanically extracting any part of the thrombus. The absence of the additional extraction procedure minimizes risk of puncture or other damage to the blood vessel in which the thrombus is located. The absence of a thrombolytic drug minimizes or eliminates the risk of bleeding associated with the use of such thrombolytic drugs.

Where the ferrofluid droplets 100 with magnetic particles 10 are impregnated with a thrombolytic drug via carrier particles 12, or where the thrombolytic drug is otherwise impregnated into the ferrofluids via the carrier fluids or as discussed previously above, the thrombolytic drug works in combination with the vortex 110 to break-up and remove the occluding thrombus 2, ideally without need to mechanically extract any portion of the thrombus. Though the risk of bleeding is present due to the use of the thrombolytic drug in this instance, the risks attendant with the additional extraction procedure of a drugless procedure are minimized.

Of course, the artisan will appreciate that where a combination of abrasives 11 and a thrombolytic drug is used with the ferrofluid droplets 100, regardless of how the thrombolytic drugs are provided, the combination works with the vortex 110 to break-up and remove the occluding thrombus 2, ideally also without the need for mechanical extraction of the thrombus. Thus, the use of such a combination would minimize at least the risks associated with the additional extraction procedure.

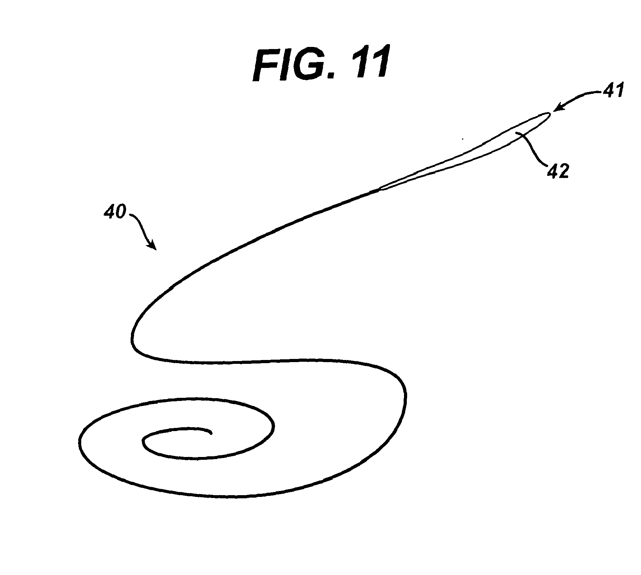

Once the occluding thrombus 2 is removed, the components of the ferrofluid that are magnetic in nature, e.g., the magnetic particles 10, the abrasives 11, if made of a magnetic material, and any carrier particles 12, if made of a magnetic material, may either remain in the patient's bloodstream, or may be magnetically recaptured and removed from the body using a catheter or micro-catheter by directing the magnetic particles 10, and other magnetic components of the ferrofluids, out of the bloodstream through the catheter or micro-catheter, as the case may be. The catheter or micro-catheter in this instance could include a guide-wire having a magnetic tip (Fig. 11), for example, that would attract the magnetic particles 10 thereto and thereby guide the ferrofluids through and out of the blood vessel 1, via the catheter or micro-catheter, as the guide-wire is extracted from the blood vessel in conventional manner through the catheter or micro-catheter.

Referring to Fig. 7, a magnetic field generator system is shown. The magnetic field generator system comprises a tubular member 20, at least one magnet 21, a collar 22, a movable table 23 and a base 24. The at least one magnet 21 is placed in the collar 22 positioned along a circumference of the tubular member 20. The collar 22 is movable or rotatable. Of course, the artisan should readily appreciate that the magnetic field may be generated by a plurality of magnets on or within the collar 22. Moreover, where provided, the plurality of magnets may be arranged on or within the collar in a variety of configurations.

As shown in Fig. 7, the collar 22 is positioned near a first end 25 of the tubular member. The collar 22 is translatable along the length L of the tubular member 20 such that the collar 22 can be positioned anywhere along the length L of the tubular member 20 between the first end 25 and a second end 26. For example, the collar 22 is shown in dashed lines about midway between the first end 25 and the second end 26 in Fig. 7 as well. Regardless of the translational position of the collar 22 along the tubular member 20, the collar 22 is rotatable or movable about the circumference of the tubular member. In this manner, the magnetic field generated by the at least one magnet 21 in the collar 22 is applied to the patient received within the tubular member 20. Furthermore, the magnetic collar may be tilted, allowing the collar to rotate about any axis within three-dimensional space.

Referring still to Fig. 7, the movable table 23 is movably mounted to a base 24. The table 23, for example, may move into and out of the tubular member 20 in order to position a patient in the tubular member 20 for appropriate orientation relative to the at least one magnet 21 and collar 22.

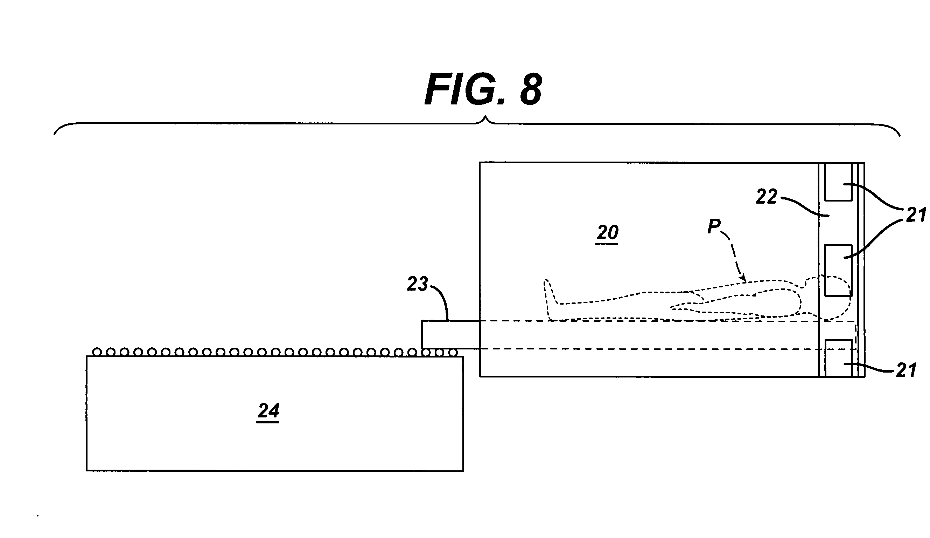

Fig. 8 illustrates more specifically a patient P positioned on the table 23 within the tubular member 20. The head of the patient P is oriented generally in line with the at least one magnet 21 in the collar 22. Such an orientation of the patient's head in this manner is contemplated when an occluding thrombus in a blood vessel of the brain is sought to be treated using the systems and methods of the invention. In practice, the collar 22 is rotated to generate, ideally, a magnetic field about the head of the patient P. The magnetic field induces the vortex 110 in the ferrofluids previously delivered to the patient. The occluding thrombus is then broken-up and removed by the ferrofluid vortex 110 as described above according to the type of ferrofluid droplets 100 used. Rotation or other movement of the collar 22 and the at least one magnet 21 is stopped, and the patient is removed from the tubular member after the thrombus has been broken-up and removed.

A conventional switch (not shown) may be used with the magnetic field generator in order to rotate or move the collar 22 when desired. Likewise, a conventional switch and moving means, such as a belt drive, rollers, glide systems or combinations thereof, may be used to move the table 23 along the base 24 and into and out of the tubular member 20 when desired. The magnetic field generator is otherwise powered by conventional means.

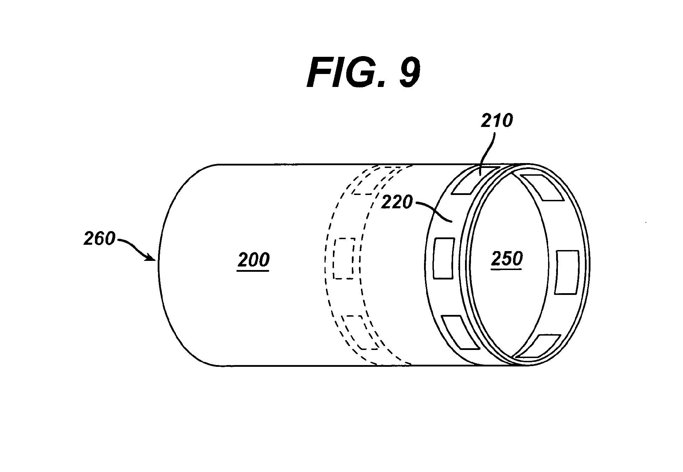

Fig. 9 illustrates a smaller scale portable magnetic field generator system according to the systems and methods of the invention. As shown in Fig. 9, the magnetic field generator comprises a tubular member 200, at least one magnet 210, and a collar 220. As in the earlier described embodiment of Figs. 1-8, the collar 220 is movable or rotatable. The tubular member 200 has a first end 250 and a second end 260. The at least one magnet 210 is placed in the collar 220 positioned along a circumference of the tubular member 200. Of course, the artisan should readily appreciate that as with the magnetic field generator of Fig. 7, the at least one magnet 210, shown as a plurality of magnets 210 in Fig. 9, could as well be comprised of a single magnetic band within the collar 220.

The magnetic field generator of Fig. 9 and operation thereof is generally the same as that described with reference to the magnetic field generator of Fig. 7 except that the tubular member 200 of Fig. 9 receives only a body part of a patient rather than the entire patient P (Fig. 8). Thus, the dimensions of the tubular member 200 and associated components are diminished from those of the tubular member 20 and associated components of Fig. 7. The artisan should readily appreciate the dimensional differences, which are omitted herein for brevity.

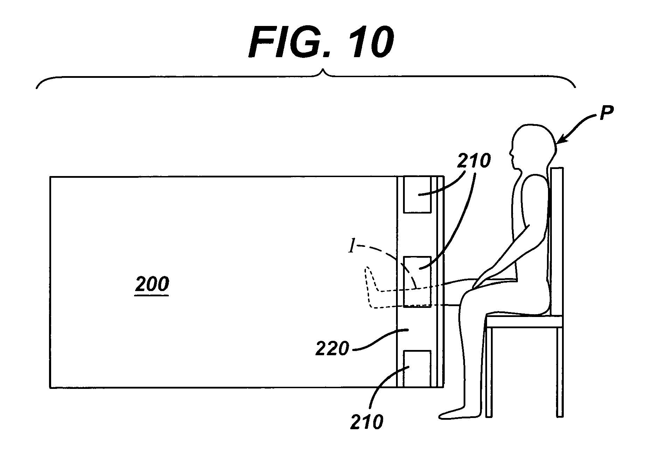

Fig. 10 illustrates a lower limb 1 of a patient P being received within the tubular member 200 of the magnetic field generator of Fig. 9. Such lower limb 1 would be received within the tubular member 200 when an occluding thrombus, such as a deep vein thrombus, exists in the lower limb and after the ferrofluids have been delivered to the patient as described above with one notable difference, namely, the ferrofluids may be delivered into larger vessels using larger catheters, as opposed to the micro-catheters discussed earlier, or may be delivered by direct puncture of the vessel with a needle connected to a syringe or other source containing the ferrofluids. The collar 220 and the at least one magnet 210 are then rotated or moved to induce the vortex 110 in the ferrofluids within the blood vessel whereat the occluding thrombus is located. The thrombus is thus broken-up and removed by the vortex 110 in a manner consistent with the type of ferrofluids used. As before, the magnetic field may instead induce high velocity jets or other movement of the ferrofluids to break-up and remove the occluding thrombus, although description of the magnetic field induced vortex is described in greater detail herein. The magnetic field is then terminated. After break-up and removal of the thrombus, the ferrofluids either remain in the bloodstream for eventual consumption by naturally occurring phagocytotic cells, or may have the magnetic components of the ferrofluids, e.g, any magnetic particles, magnetic abrasive particles or other magnetic components, magnetically re-captured and removed from the bloodstream using a magnetic tipped guide-wire and catheter or micro-catheter as discussed above with respect to other embodiments described herein. Rotation or other movement of the collar 220 and the at least one magnet 210 is stopped, and the patient's body part is removed from the tubular member after the occluding thrombus has been broken-up and removed.

Ideally, the portable magnetic field generator is transportable using a transport device, such as a conventional dolly-like apparatus, for example, similar to the manner in which oxygen tanks are commonly transported. Preferably, the transport device would include a power supply system to which the magnetic field generator could be connected. Of course, the artisan should readily appreciate that, where provided, the power supply system would provide sufficient power to rotate or otherwise move the collar 220 and the at least one magnet 210 and, where collar 220 contains electromagnets as opposed to permanent magnets, to generate a magnetic field of sufficient gradient to induce the movement of the ferrofluids needed to break-up and remove the occluding thrombus. Alternatively, the portable magnetic field generator could be powered by other conventional non-portable means.

The artisan should appreciate that in any of the embodiments of the magnetic field generator described herein, the at least one magnet may a permanent magnet, or may be an electromagnet, as the source of the magnetic field. Where the at least one magnet is a permanent magnet, power may be required only to rotate or move the collar 22 or 220, as the case may be. Alternatively, where a portable magnetic generator is used having at least one permanent magnet in the collar, rotation or movement of the collar may instead be achieved by manual means. In any event, a position of the at least one magnet, or a position of the patient relative to the at least one magnet, may be altered in order to alter the strength, geometry or gradient of the magnetic field generated thereby.

Further alternatively, the collar 22 or 220, as the case may be, may instead include a plurality of electromagnets located along the circumference of the collar, similar to as shown in Figs. 7 and 9. The electromagnets may be on or within the collar. Changes in the strength and geometry of the magnetic field generated in this instance by the magnetic field generator may be achieved by individually tuning the strength of the individual electromagnets in the collar, or by sequencing their operation so that some of the electromagnets are on, while other electromagnets are off at any given time. This arrangement of the magnetic field generator would allow the collar to remain stationary and not rotate, even as a sufficiently changing magnetic field occurred to induce the vortex 110, as otherwise discussed above.

Fig. 11 illustrates a guide-wire 40 for use with a conventional catheter or micro-catheter according to the systems and methods of the invention. The guide-wire 40 comprises at a magnetic tip 41 and at least one sensor 42 at its tip, but is otherwise conventional. The guide-wire 40 that has the at least one sensor 42 may be inserted through the catheter or micro-catheter with the ferrofluid droplets 100 to the site of the occluding thrombus. Alternatively, the tip of the catheter or micro-catheter may be provided with the at least one sensor 42. In any event, the at least one sensor 42 may be used to measure pressure, temperature, guide-wire tip deflection, or other conditions occurring at the occluding thrombus site which might indicate how the ferrofluids are performing. For example, where the at least one sensor 42 is a tip deflection sensor, the sensor 42 will detect the amount or angle of deflection of the guide-wire tip 41 within the blood vessel, wherein the bending or deflection of the guide-wire tip indicates the direction and rotation of the magnetic field generated by the magnetic field generator of Fig. 7 or Fig. 9. If the amount or degree of angular deflection of the tip 41 is insufficient, one can presume that the intended vortex 110 of the ferrofluids is not being created by the generated magnetic field. The strength, geometry, or gradient of the magnetic field can thus be altered, or the position of the patient can be altered to more appropriately align the site of the occluding thrombus with the magnets, and hence the magnetic field, of the magnetic field generator. Where provided, the magnetic tip 41 of the guide-wire 40 is also usable to magnetically attract the magnetic particles in the colloid of ferrofluids and remove these particles from the bloodstream of the patient after the occluding thrombus has been removed, as discussed earlier. Alternatively, the at least one sensor may be omitted.

The various exemplary embodiments of the invention as described hereinabove do not limit different embodiments of the present invention. The material described herein is not limited to the materials, designs, or shapes referenced herein for illustrative purposes only, and may comprise various other materials, designs or shapes suitable for the systems and procedures described herein as should be appreciated by one of ordinary skill in the art.

## Claims

1. A system for treating an occluding thrombus in a blood vessel, the system comprising:
magnetically manipulable ferrofluids disposed within the blood vessel; and
a magnetic field generator, the ferrofluids being manipulated to remove the occluding thrombus from the blood vessel according to a magnetic field generated by the magnetic field generator.

2. The system of claim 1, wherein the ferrofluids further comprises magnetic particles dispersed in a carrier fluid within the ferrofluids.

3. The system of claim 2, wherein the carrier fluid is one of a water-based, a water-alcohol-based, or a hydrocarbon-based carrier fluid.

4. The system of claim 3, wherein the carrier fluid is hydrophilic.

5. The system of claim 3, wherein the carrier fluid is hydrophobic.

6. The system of claim 3, wherein the ferrofluids further comprises abrasive particles mixed into the ferrofluids, the abrasive particles displaceable to a surface of the ferrofluids when exposed to a magnetic field.

7. The system of claim 4, wherein the ferrofluids further comprise hydrophobic abrasive particles mixed into the ferrofluids, the hydrophobic abrasive particles displaceable to a surface of the ferrofluids when subject to the hydrophilic carrier fluid.

8. The system of claim 5, wherein the ferrofluids further comprise hydrophilic abrasive particles mixed into the ferrofluids, the hydrophilic abrasive particles displaceable to a surface of the ferrofluids when subject to the hydrophobic carrier fluid.

9. The system of claim 3, wherein the ferrofluids further comprise a thrombolytic drug.

10. The system of claim 9, wherein the thrombolytic drug is bonded to one of the magnetic particles or the abrasive particles, is mixed into the carrier fluids, or is added to the ferrofluids by carrier particles to deliver the thrombolytic drug throughout the ferrofluids.

11. The system of claim 2, wherein the ferrofluids further comprise a combination of abrasive particles and a thrombolytic drug carried with the ferrofluids.

12. The system of claim 1, further comprising:
a catheter or micro-catheter for delivering the ferrofluids directly to a site of the occluding thrombus within the bloodstream.

13. The system of claim 12, further comprising at least one sensor on or near a tip of the catheter or micro-catheter for determining the activity of the ferrofluids at the thrombus site.

14. The system of claim 12, further comprising:
a guide-wire having a magnetic tip.

15. The system of claim 14, further comprising;
at least one sensor on the tip of one of the catheter, the micro-catheter, or the guide-wire for determining the activity of the ferrofluids at the site of the occluding thrombus.

16. The system of claim 15, wherein one of the at least one sensor is a deflection sensor to determine the amount of deflection of the guide-wire tip as an indication of the activity of the ferrofluids at the site of the occluding thrombus.

17. The system of claim 16, wherein the at least one sensor further comprises a temperature or pressure sensor.

18. The system of claim 17, wherein a strength, geometry or gradient of the magnetic field within the blood vessel is determined based on the data sensed from the at least one sensor.

19. The system of claim 1, wherein the magnetic field generator further comprises:
a tubular member having a first end and a second end;
a collar positionable along a circumference of the tubular member between the first end and the second end of the tubular member;
at least one magnet provided with the collar;
a movable table movably mounted to a base, the movable table oriented to hold a human patient and movable into and out of the tubular member with the patient aboard said table; and
means for powering the movable table and the collar.

20. The system of claim 1, wherein the magnetic field generator further comprises:
a tubular member having a first end and a second end;
a collar positionable along a circumference of the tubular member between the first end and the second end of the tubular member;
at least one magnet provided with the collar; and
means for powering the collar, wherein only a body part of a patient is received within the tubular member.

21. The system of claim 19 or 20, wherein the collar is rotatable or movable.

22. The system of claim 19 or 20, wherein the at least one magnet is a permanent magnet.

23. The system of claim 19 or 20, wherein the at least one magnet is an electromagnet.

24. The system of claim 1, further comprising means for intravenously delivering the ferrofluids to the bloodstream.

25. The system of claim 24, wherein the ferrofluids are concentrated to a site of the occluding thrombus prior to the removal of the occluding thrombus by the ferrofluids.

26. The system of claim 1, wherein the ferrofluids form a vortex, high-velocity jets or other motion for breaking-up the occluding thrombus upon manipulation by the magnetic field.

27. The system of claim 14, wherein the guide-wire with the magnetic tip further comprises a magnetic re-capture device for re-capturing and directing magnetic components within the ferrofluids through the catheter or micro-catheter to remove the magnetic components of the ferrofluids from the blood vessel.
